Europäisches Patentamt

European Patent Office

Office européen des brevets

(19)

(11) Veröffentlichungsnummer: **0 003 303**
**B1**

(12) **EUROPÄISCHE PATENTSCHRIFT**

(21) Anmeldenummer: **79100104.3**

(22) Anmeldetag: **15.01.79**

(51) Int. Cl.³: **C 07 C 85/24,**
**C 07 C 87/50**

(54) Verfahren zur Herstellung von an ortho-Isomeren reichen Polyaminen der Diphenylmethanreihe

(30) Priorität: **25.01.78 DE 2803126**

(43) Veröffentlichungstag der Anmeldung:
**08.08.79 Patentblatt 79/16**

(45) Bekanntmachung des Hinweises auf die Patenterteilung:
**17.09.80 Patentblatt 80/19**

(84) Benannte Vertragsstaaten:
**BE DE FR GB IT NL**

(56) Entgegenhaltungen:
**DE - A - 1 518 406**
**DE - A - 1 937 685**
**DE - A - 2 426 116**

(73) Patentinhaber: **BAYER AG**
**Zentralbereich Patente, Marken und Lizenzen**
**D - 5090 Leverkusen 1, Bayerwerk (DE)**

(72) Erfinder: **Eifler, Willi, Dr.**
**Katharinental 25**
**D - 5070 Bergisch-Gladbach (DE)**
**Ellendt, Günther, Dr.**
**Deswatinesstrasse 81**
**D - 4150 Krefeld-Bockum (DE)**

Verfahren zur Herstellung von an ortho-Isomeren reichen Polyaminen der Diphenylmethanreihe

Die vorliegende Erfindung betrifft ein verbessertes Verfahren zur Herstellung von Polyaminen der Diphenylmethanreihe, bei welchem hohe Ausbeuten an Diaminodiphenylmethanen, d.h. zweikernigen Diaminen mit hohen Ausbeuten an ortho-Isomeren, d.h. insbesondere an 2,2'- und 2,4'-Diaminodiphenylmethan einhergehen.

Die Aufgabe, Polyamingemische der Diphenylmethanreihe mit einem hohen Zweikern-Anteil und einem hohen Gehalt an ortho-Isomeren zur Verfügung zu stellen ist mit den bislang bekannt gewordenen Verfahren zu Anilin/Formadehy-Kondensation noch nicht zufriedenstellend gelöst worden. So gestattet zwar beispielsweise das Verfahren der DE—OS 1 937 685 die Herstellung von an ortho-Isomeren reichen Polyaminen, jedoch liegt der Gehalt der Polyamingemische der Ausführungsbeispiele dieser OS an Diaminen stets deutlich unter 80%. Außerdem ist beim Verfahren der gennanten OS ein Erhitzen des zunächst aus Anilin und Formaldehyd gebildeten Aminals auf mindestens 125°C erforderlich, was eine aufwendige Entfernung auch der letzten Feuchtigkeitsspuren zur Voraussetzung hat.

Es ist zwar aus der DE—OS 1 518 406 grundsätzlich bekannt, daß der Diaminanteil von Anilin/Formaldehyd-Kondensaten vom Molverhältnis Anilin zu Formaldehyd abhängt und um so höher liegt je größer der Anilinüberschuß ist. Diese Erkenntnis allein genügt jedoch ebensowenig wie die kombinierte Lehre der letztgenannten beiden Offenlegungsschriften, um zu einem Verfahren zu gelangen, welches gleichzeitig eine optimale Ausbeute an Diaminen und eine optimale Ausbeute an ortho-Isomeren gestattet.

Auf den Erkenntnissen der genannten Vorveröffentlichungen aufbauend wurde nunmehr gefunden, daß durch die nachstehend beschriebene erfindungswesentliche Maßnahme Anilin/Formaldehyd-Kondensate zugänglich sind, die bezüglich ihres Gehalts an ortho-Isomeren den Produkten der DE—OS 1 937 685 zumindest vergleichbar und bezüglich ihres Gehalts an Diaminen den Produkten der Vorveröffentlichung überlegen sind, wobei das erfindungsgemäße Verfahren im übrigen ohne aufwendiges Abdestillieren auch der letzten Wasserspuren aus dem aus Anilin und Formaldehyd gebildeten Aminal durchgeführt werden kann. Das erfindungsgemäße Verfahren gestattet im übrigen die Herstellung von Polyaminen, deren Isomerenverteilung bei hohem Diamingehalt in weiten Grenzen eingestellt werden kann.

Gegenstand der vorliegenden Erfindung ist ein Verfahren zur Herstellung von an ortho-Isomeren reichen Polyaminen der Diphenylmethanreihe durch Umsetzung von Anilin mit Formaldehyd zu N-substituierten Vorkondensaten, Entfernung des bei dieser Reaktion gebildeten, sowie des gegebenenfalls in Form wäßriger Formaldehydlösung eingebrachten Wassers, Umlagerung der Vorkondensate in Gegenwart von Anilinsalzen starker Säuren zu den entsprechenden am aromatischen Kern substituierten primären Aminen, Neutralisation des verwendeten Katalysators und weitere an sich bekannte Aufarbeitung der so erhaltenen Umsetzungsprodukte, wobei die Mengen der Reaktionspartner und der genannten starken Säuren so bemessen werden, daß die Gesamtmenge des zum Einsatz gelangenden Anilins einem Anilin/Formaldehyd-Molverhältnis von 4:1 bis 20:1 und die Gesamtmenge der mitverwendeten starken Säuren bezogen auf die Gesamtmenge des zum Einsatz gelangenden Anilins einem Anilin/Säure-Äquivalentverhältnis von 10:1 bis 1000:1 entspricht, dadurch gekennzeichnet, daß man mindestens die Hälfte der Gesamtmenge des zum Einsatz gelangenden Anilins mit der Säure zu einem Anilin/Anilinsalz-Gemisch vereinigt dieses Gemisch auf eine Temperatur von 100—200°C erhitzt und das auf einer Temperatur von 0 bis 100°C gehaltene N-substituierte Vorkondensat in dieses Gemisch einträgt und anschließend die Umlagerung des Vorkondensats bei einer Temperatur im Temperaturbereich zwischen 100 und 250°C zu Ende führt.

Ausgangsmaterialien für das erfindungsgemäße Verfahren sind Anilin, und Formaldehyd.

Im Prinzip ist es jedoch auch möglich andere aromatische Amine wie z.B. o- und m-Toluidin, o- und m-Anisidin, o- und m-Phenetidin, N-Methylanilin, N-Äthylanilin, 2,4 Diaminotoluol, sowie beliebige Gemische derartiger Amine anstelle des Anilins einzusetzen.

Der Formaldehyd kommt vorzugsweise in Form wäßriger Formaldehydlösungen zum Einsatz. Selbstverständlich können auch beliebige unter den Reaktionsbedingungen Formaldehyd-abspaltende Substanzen verwendet werden.

Bei den erfindungsgemäß zum Einsatz gelangenden Katalysatoren handelt es sich um wasserlösliche Säuren mit einem unter 2,5, vorzugsweise unter 1,5 liegenden pKa-Wert. Beispiele hierfür sind Salzsäure, Bromwasserstoffsäure, Schwefelsäure, Trifluoressigsäure, Methansulfonsäure, Trifluormethansulfonsäure, Benzolsulfonsäure oder Phosphorsäure.

Bevorzugt eingesetzter Katalysator ist Salzsäure. Die genannten Säuren können auch im Gemisch mit sauren oder neutralen Salzen derartiger Säuren wie z.B. den entsprechenden Ammoniumsalzen oder auch den entsprechenden Alkalisalzen eingesetzt werden, obwohl dies weniger bevorzugt ist.

Bei den zur Neutralisation der Säuren einzusetzenden Basen handelt es sich vorzugsweise um wäßrige anorganische Basen wie beispielsweise Natronlauge.

Zur Durchführung des erfindungsgemäßen Verfahrens wird zunächst in Abwesenheit der genannten Katalysatoren aus dem Anilin und dem Formaldehyd ein N-substituiertes Vorkondensat, d.h. Aminal hergestellt. Diese Reaktion wird im Temperaturbereich zwischen 0 und 100°C vorzugsweise zwischen 30 und 80°C nach an sich bekannten Verfahren des Standes der Technik durchgeführt. Das dabei entstehende Kondensationswasser, sowie das gegebenenfalls in Form wäßriger Formalinlösung eingebrachte Wasser wird im Anschluß an die Aminalbildung durch Phasentrennung gegebenenfalls auch unter anschließender Restwasserentfernung z.B. durch Vakuumdestillation entfernt. Im allgemeinen ist eine derartige Restwasserentfernung nach erfolgter Phasentrennung nicht erforderlich.

Zu dieser Vorkondensation gelangen die Reaktionspartner in einem Anilin/Formaldehyd-Molverhältnis von mindestens 2:1 entsprechenden Mengen zum Einsatz. Das Verhältnis zwischen der Gesamtmenge des beim erfindungsgemäßen Verfahren eingesetzten Anilins, d.h. inklusive dem erst in der zweiten Stufe zusammen mit dem Säurekatalysator eingesetzten Anilin zur Formaldehydmenge entspricht im übrigen einem Molverhältnis Anilin: Formaldehyd von 4:1 bis 20:1 vorzugsweise 8:1 bis 20:1, wobei höchstens die Hälfte der Gesamtmenge des Anilins bereits in der ersten zum Aminal führenden Reaktionsstufe eingesetzt wird. Dies bedeutet, daß mindestens die Hälfte der Gesamtmenge des Anilins erst im zweiten Reaktionsschritt eingesetzt wird.

Das in der ersten Reaktionsstufe gewonnene Aminal bzw. dessen Lösung in gegebenenfalls vorliegendem überschüssigem Anilin weist vor der zweiten Reaktionsstufe, d.h. vor der Vereinigung mit den Anilinsalz-Katalysatoren eine Temperatur im Bereich von 0 bis 100°C, vorzugsweise von 30 bis 80°C auf.

In der nächsten Reaktionsstufe des erfindungsgemäßen Verfahrens erfolgt nun die Vereinigung des Aminals bzw. dessen Lösung in überschüssigem Anilin mit einem Anilin-Säure-Gemisch, welches durch Teilneutralisation einer zweiten Anilinmenge mit einem der beispielhaft genannten Säure-katalysatoren erhalten worden ist.

Bei dieser Teilneutralisation kommt die Säure in solchen Mengen zum Einsatz, die einem Protonierungsgrad bezogen auf alle im Reaktionsgemisch vorliegende Aminstickstoffatome von 0,1 bis 10 vorzugsweise 0,1 bis 5% entsprechen. In diese Bilanz sind alle vorliegenden Aminstickstoffatome, d.h. inklusive diejenigen des Aminals, sowie diejenigen des bei der Aminalbildung im Überschuß eingesetzten Anilins, sowie diejenigen des zusammen mit der Säure eingesetzten Anilins einzubeziehen. Unter "Protonierungsgrad" ist hierbie der Prozentsatz der in Form vom Ammoniumgruppen, d.h. "protoniert" vorliegenden Aminstickstoffatome bezogen auf Gesamtmenge der Aminstickstoffatome zu verstehen.

Bei der Vereinigung des teilneutralisierten Anilins mit der Aminal-Komponente wird erstere auf einer Temperatur im Bereich von 100 bis 200°C vorzugsweise zwischen 100 und 150°C gehalten.

Nach der Vereinigung der beiden Komponenten erfolgt die Umlagerung des Aminals zum kernsubstituierten Polyamin durch Erhitzen des Reaktionsgemischs auf 100—250°C vorzugsweise 120—200°C während eines Zeitraums von ca. 5 bis 300 Minuten.

Die Wahl der Temperatur für die Umlagerung hängt im wesentlichen vom gewählten Anilin/Katalysator-Molverhältnis und von der eingestellter Verweilzeit für die Umlagerung ab. Bei kleinem Molverhältnis und langen Verweilzeiten kann die Umlagerungstemperatur an der unteren Grenze des infrage kommenden Bereichs von 100—250°C gewählt werden, während umgekehrt und zwar besonders bei hohen Anilin/Katalysator-Molverhältnissen hohe Umlagerungstemperaturen zweckmäßig sind. Die bei erfindungsgemäßen Verfahren hauptsächlich angewendeten Temperaturen liegen zwischen 120—200°C bei Atmosphärendruck oder Überdruck.

Das erfindungsgemäße Verfahren kann selbstverständlich kontinuierlich und diskontinuierlich betrieben werden, wobei man sich in beiden Fällen der an sich bekannten Apparaturen und Vorrichtungen bedient. So kann beispielsweise die kontinuierliche Durchführung des erfindungsgemäßen Verfahrens folgendermaßen erfolgen:

Gemessene und geregelte Ströme aus Anilin und wäßriger Formalinlösung werden in einem kontinuierlich zu betreibenden Reaktor innerhalb des obengenannten Temperaturbereichs zum N-substituierten Aminal umgesetzt. Die dabei freiwerdende Wärme kann im Reaktionsgemisch verbleiben oder aber auch durch Wärmeaustausch abgeführt werden. Das entstehenden zweiphasige Reaktionsgemisch wird in einem kontinuierlich zu betreibenden Phasenabscheider getrennt. Die obere, wäßrige Phase wird der Abwasseraufarbeitung zugeführt. Die untere, organische Phase wird in einem weiteren kontinuierlich zu betreibenden Reaktor mit einem gemessenen und geregelten Strom eines Anilin/Salzsäure-Gemisch vereinigt, wobei das letztgenannte Gemisch bezüglich seiner Zusammensetzung und seiner Temperatur den obengemachten Ausführungen entspricht. Die Umlagerungsreaktion erfolgt dann anschließend in mehreren, hintereinander geschalteten Reaktoren, deren Temperaturen innerhalb des für die Umlagerungsreaktion obengenannten Temperaturbereichs liegen. Das gegebenenfalls im Temperaturbereich über 100°C verdampfende Wasser oder Wasser/Anilin-Azeotrop wird in den Reaktor zur Aminalbildung oder in den nachgeschalteten Abscheider geleitet. Anschließend erfolgt die an sich bekannte Aufarbeitung des Reaktionsgemisches, die im wesentlichen in der Neutralisation des Katalysators und der destillativen Aufarbeitung der Verfahrensprodukte besteht.

Die nach dem erfindungsgemäßen Verfahren erhaltenen Produkte können nach den bekannten Methoden des Standes der Technik mit Phosgen zu Isocyanaten umgesetzt werden. Darüber hinaus eignen sich die Verfahrensprodukte zur Verwendung als Vernetzer für Epoxide oder für modifizierte Isocyanate.

Folgende Beispiele erläutern die Erfindung. Die dabei verwendete Salzsäure und das Formalin sind beide 30%ige wäßrige Lösungen.

### Beispiel 1 (Vergleich)

558 g (6 Mol) Anilin und 200 g (2 Mol) Formalin werden bei Zimmertemperatur unter Rühren zusammengegeben. Die Temperatur steigt auf 65°C. Man rührt 30 Minuten bei 65°C nach und trennt die Phasen bei 65°C. Die organische Phase wird in eine auf 40°C gebrachte Mischung aus 1302 g (14 Mol) Anilin und 40,6 g (0,33 Mol) Salzsäure gegeben. Man erhitzt zum Sieden und hält 4 Stunden am Rückfluß bei 128°C. Man neutralisiert mit 350 ml 6%iger Natronlauge. Die abgeschiedene organische Phase wird im Wasserstrahlvakuum bis zu einer Kopftemperatur von 220°C erhitzt. Der Destillationsrückstand stellt das Verfahrensprodukt dar. Es enthält 89,1% Diamine.

Die gaschromatographisch ermittelte Isomerenverteilung ist in der Tabelle enthalten.

### Beispiele 2 (Vergleich) und 3 (erfindungsgemäß)

Man verfährt analog Beispiel 1 mit dem Unterschied, daß die Anilin/Salzsäuremischung nicht bei 40°C sondern bei 80°C bzw. 120°C vorgelegt wird. Analysen siehe Tabelle.

### Beispiele 4—9

Man verfährt analog Beispiel 1 nach den in der Tabelle gemachten Angaben. Analysen siehe Tabelle. Erfindungsgemäß sind die Beispiele 7 und 8 mit einer mindestens bei 100°C liegenden Temperatur der Anilin/Salzsäure-Mischung ausgeführt worden.

### Beispiel 10

Man verfährt analog Beispiel 1 und den in der Tabelle gemachten Angaben mit dem Unterschied, daß die Rückflußtemperatur des sauren Reaktionsgemisches durch Abdestillieren von Wasser auf 180°C angehoben wird. Nach 60 Min. bei 180°C verfährt man weiter wie in Beispiel 1 beschrieben. Analyse siehe Tabelle.

### Beispiele 11—12

Man verfährt analog Beispiel 10 mit dem Unterschied, daß der Siedepunkt des sauren Reaktionsgemisches durch Abdestillieren von Wasser auf 185°C angehoben wird. Bei dieser Temperatur hält man dann 90 Min. bzw. 180 Min. Analysen siehe Tabelle.

### Tabelle

| Beispiel | Vorreaktion Anilin g | Vorreaktion Formalin g | Saurer Teil Anilin g | Saurer Teil Salzsäure g | Temperatur der Anilin/Salzsäure-mischung | Diaminanteil % | 2,2'- Diamino-diphenylmethan % | 2,4'- % | 4,4'- % |
|---|---|---|---|---|---|---|---|---|---|
| 1 | 558 | 200 | 1302 | 40,6 | 40°C | 89,1 | 4,2 | 23,9 | 71,7 |
| 2 | 558 | 200 | 1302 | 40,6 | 80°C | 87,8 | 5,3 | 27,7 | 66,9 |
| 3 | 558 | 200 | 1302 | 40,6 | 120°C | 86,8 | 7,3 | 32,5 | 59,9 |
| 4 | 372 | 200 | 1488 | 40,6 | 30°C | 89,0 | 4,5 | 24,3 | 71,0 |
| 5 | 930 | 200 | 930 | 40,6 | 30°C | 89,6 | 3,2 | 22,1 | 74,5 |
| 6 | 427,8 | 200 | 37,2 | 40,6 | 40°C | 62,7 | 1,1 | 15,1 | 83,6 |
| 7 | 558 | 200 | 2232 | 60,8 | 130°C | 91,0 | 7,5 | 37,0 | 55,4 |
| 8 | 465 | 200 | 3255 | 40,6 | 145°C | 91,9 | 12,2 | 38,3 | 49,4 |
| 9 | 465 | 200 | 3255 | 40,6 | 40°C | 93,2 | 7,1 | 28,3 | 64,5 |
| 10 | 446 | 200 | 1786 | 11,7 | 100°C | 86,7 | 11,4 | 31,1 | 57,2 |
| 11 | 465 | 200 | 2325 | 7,3 | 130°C | 88,2 | 16,8 | 35,8 | 47,4 |
| 12 | 465 | 200 | 2325 | 3,65 | 130°C | 88,0 | 18,7 | 36,0 | 45,0 |

**Patentanspruch**

Verfahren zur Herstellung von an ortho-Isomeren reichen Polyaminen der Diphenylmethanreihe durch Umsetzung von Anilin mit Formaldehyd zu N-substituierten Vorkondensaten, Entfernung des bei dieser Reaktion gebildeten, sowie des gegebenenfalls in Form wäßriger Formaldehydlösung eingebrachten Wassers, Umlagerung der Vorkondensate in Gegenwart von Anilinsalzen starker Säuren zu den entsprechenden am aromatischen Kern substituierten primären Aminen, Neutralisation des verwendeten Katalysators und weitere an sich bekannte Aufarbeitung der so erhaltenen Umsetzungsprodukte, wobei die Mengen der Reaktionspartner und der genannten starken Säuren so bemessen werden, daß die Gesamtmenge des zum Einsatz gelangenden Anilins einem Anilin/Formaldehyd-Molverhältnis von 4:1 bis 20:1 und die Gesamtmenge der mitverwendeten starken Säure bezogen auf die Gesamtmenge des zum Einsatz gelangenden Anilins einem Anilin/Säure-Äquivalentverhältnis von 10:1 bis 1000:1 entspricht, dadurch gekennzeichnet, daß man mindestens die Hälfte der Gesamtmenge des zum Einsatz gelangenden Anilins mit der Säure zu einem Anilin/Anilinsalz-Gemisch vereinigt dieses Gemisch auf eine Temperatur von 100—200°C erhitzt und das auf einer Temperatur von 0 bis 100°C gehaltene N-substituierte Vorkondensat in dieses Gemisch einträgt und anschließend die Umlagerung des Vorkondensats bei einer Temperatur im Temperaturbereich zwischen 100 und 250°C zu Ende führt.

**Revendication**

Procédé de préparation de polyamines de la série due diphénylméthane, riches en ortho-isomères par réaction d'aniline avec du formaldéhyde pour former des précondensats N-substitués, par élimination de l'eau formée lors de cette réaction, ainsi que de l'eau éventuellement introduite sous forme d'une solution aqueuse de formaldéhyde, par transposition des précondensats, en présence de sels d'aniline d'acides forts, en amines primaires correspondantes substituées sur le noyau aromatique, par neutralisation due catalyseur utilisé et par un traitement complémentaire connu en soi des produits réactionnels ainsi obtenus, les quantités des partenaires réactionnels et des acides forts mentionnés étant calculées de telle sorte que la quantité totale de l'aniline utilisée corresponde à un rapport molaire aniline/formaldéhyde de 4:1 à 20:1 et que la quantité totale (rapportée à la quantité totale de l'aniline utilisée) des acides forts utilisés conjointement corresponde à un rapport d'équivalents d'aniline/acide de 10:1 à 1.000:1, caractérisé en ce qu'on combine au moins la moitié de la quantité totale de l'aniline utilisée avec l'acide en un mélange d'aniline/sel d'aniline, on chauffe ce mélange à une température de 100—200°C et on introduit, dans ce mélange, le précondensat N-substitué maintenu à une température de 0 à 100°C, puis on achève la transposition du précondensat à une température allant de 100 à 250°C.

**Claim**

A process for the preparation of polyamines of the diphenyl methane series which are rich in ortho isomers, by reacting aniline with formaldehyde to produce N-substituted precondensates; removing the water formed in this reaction as well as any water introduced in the form of aqueous formaldehyde solution; rearranging the pre-condensate in the presence of aniline salts of strong acids to the corresponding primary amines substituted on the aromatic nucleus; neutralizing the catalyst used; and subsequently working up in known manner the resulting reaction products; the quantities of reactants and of the aforesaid strong acids used being calculated so that the total quantity of aniline used corresponds to an aniline/formaldehyde molar ratio of from 4:1 to 20:1, and the total quantity of strong acid added, based on the total quantity of aniline used, corresponds to an aniline/acid equivalent ratio of from 10:1 to 1,000:1, characterised in that at least half the total quantity of aniline used is combined with the acid to form an aniline/aniline-salt mixture; the mixture is heated to a temperature of from 100 to 200°C, the N-substituted pre-condensate, which has been maintained at a temperature of 0 to 100°C, is introduced into this mixture; and rearrangement of the pre-condensate is then completed at a temperature within the range of from 100 to 250°C.